(19) Europäisches Patentamt / European Patent Office / Office européen des brevets

(11) EP 2 146 690 B1

(12) **EUROPEAN PATENT SPECIFICATION**

(45) Date of publication and mention of the grant of the patent:
**21.03.2018 Bulletin 2018/12**

(21) Application number: **08799796.1**

(22) Date of filing: **14.04.2008**

(51) Int Cl.:
***A61K 9/08*** *(2006.01)* ***A61K 9/14*** *(2006.01)*
***A61K 31/55*** *(2006.01)*

(86) International application number:
**PCT/US2008/060251**

(87) International publication number:
**WO 2008/128191 (23.10.2008 Gazette 2008/43)**

(54) **ORAL CEPHALOTAXINE DOSAGE FORMS**

ORALE DOSIERUNGSFORMUNGEN VON CEPHALOTAXIN

FORMES DOSIFIEES ORALES DE CEPHALOTAXINE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **13.04.2007 US 923446 P**

(43) Date of publication of application:
**27.01.2010 Bulletin 2010/04**

(73) Proprietor: **Teva Pharmaceuticals International GmbH**
**8645 Jona (CH)**

(72) Inventors:
- **BROWN, Dennis**
**Menlo Park, CA 94025 (US)**
- **MICHAELS, Shawnya**
**El Granada, CA 94018 (US)**

(74) Representative: **Towler, Philip Dean**
**Dehns**
**St Bride's House**
**10 Salisbury Square**
**London EC4Y 8JD (GB)**

(56) References cited:
**US-A1- 2004 082 565**

- **VISANI C ET AL: "Effects of homoharringtonine alone and in combination with alpha interferon and cytosine arabinoside on in vitro growth and induction of apoptosis in chronic myeloid leukemia and normal hematopoietic progenitors" 19970101, vol. 11, 1 January 1997 (1997-01-01), pages 624-628, XP008000008**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 146 690 B1

Printed by Jouve, 75001 PARIS (FR)

## Description

### FIELD OF THE INVENTION

Pharmaceutical compositions comprising oral dosage forms of cephalotaxine and methods of administration using such oral dosage forms for preventing or treating cephalotaxine sensitive diseases disorders, conditions and syndromes are disclosed.

### BACKGROUND OF THE INVENTION

Homoharringtonine (HHT) is a cephalotaxine that has been used to treat various forms of cancer. For the most part, HHT has been administered intravenously or subcutaneously. The difficulties associated with such administration, including patient compliance are well known. US 2004/082565 discloses compositions and methods for the treatment of patients with cephalotaxines, including homoharringtonine.

### SUMMARY OF THE INVENTION

Oral dosage forms comprising a therapeutically effective amount of a cephalotaxine and a carrier selected from the group consisting of an oral dosage form comprising a therapeutically effective amount of a cephalotaxine and a pharmaceutically acceptable carrier selected from the group consisting of protein, lipid or combinations thereof;
wherein said protein is selected from the group consisting of sodium caseinate, gelatin, casein, soy protein (isolated), brown algae protein, red algae protein, whey protein concentrate, bakers yeast extract and albumin;
wherein said lipid is selected from the group consisting of coconut oil (refined), oleic acid, soybean oil (hydrogenated), aluminum palmitate, dilauryl thiodipropionate, enzyme-modified lecithin, calcium stearate, enzyme-modified fats, glyceryl palmitostereate, lecithin, mono- and diglycerides, rapeseed oil and vegetable oil. The oral dosage form can be a formulated as solutions; suspensions; capsules, including hard shell capsules and soft shell capsules; tablets, including gastric disintegrating, orally administrable, effervescent and modified release tablets; granules; powders; gels; orally administrable films; and other formulations known in the art. In some embodiments, the oral dosage form includes an enteric coating.

In a preferred embodiment, the cephalotaxine comprises homoharringtonine (cephalotaxine, 4-methyl-2-hydroxy-2-(4-hydroxy-4-methyl pentyl) butanediocate ester. Homoharringtonine is also known by its USAN designation of omacetaxine. In a further preferred embodiment, the cephalotaxine comprises a cephalotaxine analog.

Methods of treating or preventing cephalotaxine sensitive diseases, such as angiogenic diseases using oral dosage forms containing cephalotaxine are also disclosed. The angiogenic disease may be cancer, which may be characterized by microtumors or micrometastatic cancer cells. The angiogenic disease may be an angiogenic disease other than cancer, such as an inflammatory disease, including rheumatoid arthritis, osteoarthritis, asthma, and pulmonary fibrosis; diabetic retinopathy; or macular degeneration.

### BRIEF DESCRIPTION OF THE FIGURES

Figure 1 shows the general chemical structure for cephalotaxines.

Figure 2 shows the chemical structure for homoharringtonine.

Figure 3 is a plot of HHT serum concentration versus time for oral and subcutaneous administration of HHT to mice.

### DETAILED DESCRIPTION OF THE INVENTION

As used herein, the singular forms "a," "an", and "the" include plural references unless the context clearly dictates otherwise. For example, reference to "an active agent" includes a single active agent as well as two or more different active agents in combination. It is to be understood that present teaching is not limited to the specific dosage forms, carriers, or the like, disclosed herein and as such may vary.

General Definitions

As used herein, the following terms have the following meanings:

The terms "effective amount" or a "therapeutically effective amount" of a drug or pharmacologically active agent refers to a nontoxic but sufficient amount of the drug or agent to provide the desired effect. In the oral dosage forms of the present disclosure, an "effective amount" of one active of the combination is the amount of that active that is effective to provide the desired effect when used in combination with the other active of the combination. The amount that is "effective" will vary from subject to subject, depending on the age and general condition of the individual, the particular active agent or agents, and the appropriate "effective" amount in any individual case may be determined by one of ordinary skill in the art using routine experimentation.

The phrase "pharmaceutically acceptable" means moieties or compounds that are, within the scope of medical judgment, suitable for use in humans without causing undesirable biological effects such as undue toxicity, irritation, allergic response, and the like, for example.

The phrase "oral dosage form" means any pharmaceutical composition administered to a subject via the oral cavity, in which a cephalotaxine is administered, optionally with one or more additional drugs. Exemplary oral dosage forms include tablets, capsules, films, powders, sachets, granules, solutions, solids, suspensions and other formulations known in the art. An oral dosage form can be one, two, three, four, five or six units. When the oral dosage form has multiple units, all of the units are contained within a single package, (e.g. a bottle or other form of packaging such as a blister pack). When the oral dosage form is a single unit, it may or may not be in a single package. In a preferred embodiment, the oral dosage form is one, two or three units. In a particularly preferred embodiment, the oral dosage form is one unit.

The phrase "unit", as used herein, refers to the number of discrete objects to be ingested which comprise the oral dosage form. In some embodiments, the oral dosage form contains cephalotaxine within one capsule. This is a single unit, whether or not the interior of the capsule includes multiple discrete granules of active ingredient. For example, in some embodiments, the oral dosage form includes cephalotaxine and, optionally, a second drug contained within one capsule. This is also a single unit. In some embodiments, the oral dosage form includes cephalotaxine in one capsule, and a second drug in a second capsule. This is also a two unit oral dosage form, such as two capsules or tablets, and so such units are contained in a single package. Thus the term 'unit' refers to the object which the patient ingests, not to the interior components of the object.

The phrases "active ingredient", "therapeutic agent", "active", or "active agent" mean a chemical entity which can be effective in treating a targeted disorder, disease or condition.

The term "substrates" means pharmaceutically acceptable particulate materials such as beads, particles, granules, pellets, and the like, in an oral dosage form.

The term, "substantially free", as used herein, refers to a composition which contains none of the substance or less than a therapeutically effective amount of the substance for any known purpose for which the composition is intended.

The term, "essentially all", as used herein, refers to a composition in which a member selected from at least 90%, at least 93%, at least 95%, at least 98%, and at least 99% of the members of the composition have the trait which is described. For example, if "essentially all" of the acid inhibitor in a composition is enterically coated, then at least 90%, or at least 93%, or at least 95%, or at least 98%, or at least 99% of the acid inhibitor molecules of the composition are contained within an enteric coat.

## I. Cephalotaxines

The term "cephalotaxine" means alkaloids extracted from skins, stems, leaves and seeds of *Cephalotaxus fortunei* Hook and other related species, such as *Cepholotaxus sinensis* Li, *C. hainanensis* and *C. wilsoniana,* including *C. oliveri* mast *and C. harringtonia* (Powell, R.G., (1972) J. Pharm Sci., 61(8):1227-1230).

As used herein, the term cephalotaxine includes all members of that chemical family including alkaloid derivatives of the Chinese evergreen, *Cephalotaxus fortunei* and analogs thereof. The cephalotaxine family is defined by chemical structure as set forth in Figure 1.

A cephalotaxine analog is further defined but not limited to the structure depicted in Figure 1, having substituent or substitute groups at R1 and R2. Examples of R1 and/or R2 include esters, including herringtonine, isoharringtonine, homoharringtonine, deoxyharringtonine, acetylcephalotaxine and the like. Table I lists structures of R1 and R2 for some of these analogs. R1 and R2 substitutions are typically employed to improve biological activity, pharmaceutical attributes such as bioavailability or stability, or decrease toxicity. In one embodiment, R1 and/or R2 include alkyl substitutions (*e.g.*, methyl, ethyl, propyl etc.). In another embodiment, R1 and/or R2 include esters (*e.g.*, methoxy, ethoxy, butoxy, etc.). R1 and R2 are not limited to the above examples, however, in the scope of this invention.

**Table I**

| compound | R1 | R2 |
|---|---|---|
| isoharringtonine | $-OCH_3$ | 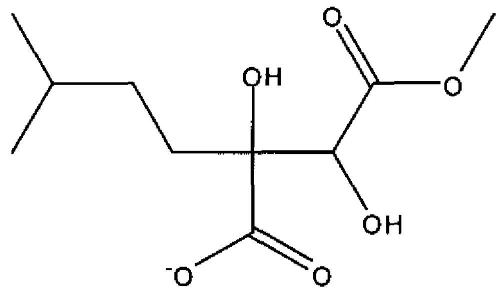 |
| harringtonine | $-OCH_3$ | 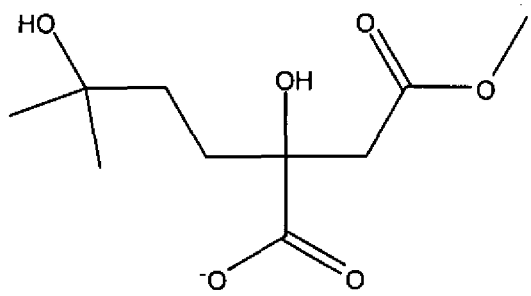 |
| acetylcephalotaxine | $-OCH_3$ | 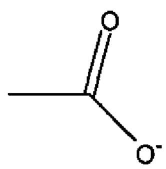 |
| homoharringtonine | $-OCH_3$ | 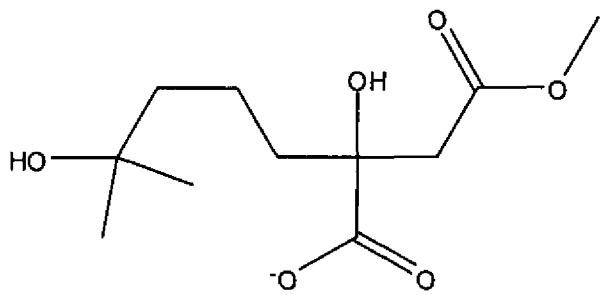 |

A specific example of cephalotaxine is homoharringtonine which is the butanediocate ester of cephalotaxine, 4-methyl-2-hydraxy-2-(4-hydraxy-4-methyl pentyl). Its chemical structure is set forth in Figure 2.

## II. Caphalotaxine Sensitive Diseases. Disorders, Conditions and Syndromes

There are a number of diseases, disorders, conditions and syndromes that are sensitive to cephalotaxines. These include angiogenic diseases and various forms of leukemia including pre-leukemic sysndromes.

### A. Angiogenic Diseases

The oral dosage form of cephalotaxine according to the invention may be orally administered to a host with an angiogenic disease. The cephalotaxine is administered in an amount sufficient to inhibit angiogenesis thereby inhibiting progression of angiogenesis and the angiogenic disease.

Angiogenesis is defined as the formation and differentiation of new blood vessels. It has been linked to a number of diseases and conditions, in particular to cancer, inflammation and certain retinal disorders. Angiogenic diseases Include, but are not limited to, solid tumors, diabetic retinopathy, inflammatory diseases (such as rheumatoid arthritis,

osteoarthritis, asthma, and pulmonary fibrosis), macular degeneration, angiofibroma , neovascular glaucoma, arteriovenous malformations, nonunion fractures, lupus and other connective tissue disorders, Osier- Weber syndrome, atherosclerotic plaques , psoriasis, corneal graft neovascularization, Pyogenic granuloma, retrolental fibroplasia, scleroderma, granulation, hemangioma, trachoma , hemophilic joints , and vascular adhesions.

Angiogenesis, the process by which new blood vessels are formed, is essential for normal body activities including reproduction, development, and wound repair. Although the process is not completely understood, it is believed to involve a complex interplay of molecules that regulate the growth of endothelial cells (the primary cells of capillary blood vessels). Under normal conditions, these molecules appear to maintain the microvasculature in a quiescent state (i.e. one of no capillary growth) for prolonged periods which may last for as long as weeks, or, in some cases, decades. When necessary (such as during wound repair), these same cells can undergo rapid proliferation and turnover within a 5 day period (Folkman, J. and Shing, Y.; J. Biol. Chem., 267(16), 10931-10934, and Folkman, J. and Klagsbrun, M. Science, 235, 442-447 (1987).

Although angiogenesis is a highly regulated process under normal conditions, many diseases (characterized as angiogenic diseases) are driven by persistent unregulated angiogenesis. Otherwise stated, unregulated angiogenesis may either cause a particular disease directly or exacerbate an existing pathological condition. For example, ocular neovacularization has been implicated as the most common cause of blindness and dominates approximately 20 eye diseases. In certain existing conditions, such as arthritis, newly formed capillary blood vessels invade the joints and destroy cartilage. In diabetes, new capillaries formed in the retina invade the vitreous, bleed, and cause blindness. Growth and metastasis of solid tumors are also dependent on angiogenesis (Folkman, J., (1986) Cancer Research, 46, 467-473, Folkman, J., (1989) J. National Cancer Institute, 82, 4-6. It has been shown, for example, that tumors that enlarge greater than 2 mm must obtain their own blood supply and do so by inducing the growth of new capillary blood vessels. Once these blood vessels become embedded in the tumor. they provide a means for the tumor to metastasize to different sites such as liver, lung or bone (Weidner. N. et al-, (1991) The New England Journal of Medicine, 324(1),1-8).

The angiogenic disease may be a disease other than a solid tumor or may be a solid tumor.

Growth and metastasis of tumors is dependent on angiogenesis. Solid tumors need oxygen and nutrients to survive and grow. Without a blood supply, potential tumors either die or remain dormant. These potential tumors can be, for example, microtumors or micrometastatic cancer cells. The "microtumors" remain as a stable cell population wherein dying cells are replaced by new cells. Microtumors may represent, for example, the initiation of a solid tumor in host that has no other solid tumors. Microtumors may also represent the remaining tumor cells present in a host after the solid tumor, from which the microtumors has metastasized, has been removed or eradicated. This condition may occur in a host that is in remission for cancerous tumors. Micrometastatic cancer cells refers to cancer cells that have not yet been vascularized to form a solid tumor.

The micro tumor becomes a rapidly growing tumor when it becomes vascularized and can expand to 16.000 times its original volume in 2 weeks after vascularization. Without the blood supply, no growth is seen (Folkman, J. (1974) Tumor Angiogenesis, Adv. Cancer Res. 19: 331 358; Ausprunk, D. H. and Folkman, J. (1977) Migration and Proliferation of Endothelial Cells in Preformed and Newly Formed Blood Vessels During Tumor Angiogenesis, Microvasc. Res. 14: 53 65.

In addition to supplying the tumor with nutrients and oxygen, angiogenesis allows the solid tumor to metastasize. The new blood vessels provide a route that enables cells from the solid tumor to migrate to other sites in the host, resulting in the formation of secondary tumors.

Thus, by inhibiting angiogenesis, the vascularization of the microtumors is minimized and the progression of metastasis and tumor growth is inhibited or stopped.

A cephalotaxine may be administered to a host with microtumors. The cephalotaxine is administered in an amount sufficient to inhibit angiogenesis thereby inhibiting growth and metastasis of the microtumors. The microtumors may represent the early onset of a disease characterized by tumor growth. The microtumors may be the result of metastasis of an established solid tumor.

Another disease characterized by excessive blood vessel growth is diabetic retinopathy. Recent studies indicate a pathogenetic role for the renin-angiotensin system (RAS) and vascular endothelial growth factor (VEGF) in the eye in response to chronic hyperglycaemia (Wilkinson-Berka J. L., et al., (2001) The Interaction Between the Renin-Angiotensin System and Vascular Endothelial Growth Factor in the Pathogenesis of Retinal Neovascularization in Diabetes, J Vase Res., 38(6):527-35).

The oral dosage form of cephalotaxine according to the invention may be administered to a host with diabetes suffering from, or at the risk of suffering from, diabetic retinopathy. The cephalotaxine is administered in an amount sufficient to inhibit angiogenesis thereby slowing progression of the diabetic retinopathy.

Angiogenesis has been implicated in chronic inflammatory diseases, including for example, rheumatoid arthritis, osteoarthritis, asthma, and pulmonary fibrosis (Walsh, D.A. and Pearson C.I. (2001), Angiogenesis in the Pathogenesis of Inflammatory Joint and Lung Diseases, Arthritis Res., (3): 147-153; Storgardl, C.M., et al., (1999), Decreased Angiogenesis and Arthritic Disease in Rabbits Treated with an v$\beta$3 Antagonist, J Clin Invest, 3(I):47-54.

The oral dosage form of cephalotaxine according to the invention may be administered to a host with an inflammatory disease. The cephalotaxine is administered in an amount sufficient to inhibit angiogenesis thereby slowing progression of the inflammatory disease. The inflammatory disease may be rheumatoid arthritis, osteoarthritis, asthma or pulmonary fibrosis.

A cephalotaxine may be administered to a host as a prophylactic treatment. By "prophylactic treatment" is meant administration of a cephalotaxine to a host to prevent the onset or progression of an angiogenic disease. An oral dosage form of cephalotaxine according to the invention may be administered to a host to prevent the onset of tumor growth or metastasis or a disease characterized by tumor growth or metastasis. Such treatment may be desirable, for example, in a host that has exhibited tumor growth, such as a cancerous tumor, but is now in remission.

A cephalotaxine may be administered to a host to prevent the onset or progression of an angiogenic disease other than cancerous tumor growth. The cephalotaxine may be administered to a host at risk of exhibiting an inflammatory disease, such as rheumatoid arthritis, osteoarthritis, asthma, or pulmonary fibrosis.

A cephalotaxine may be administered to a host that is diabetic, or at risk of becoming diabetic, as a prophylactic treatment to prevent or inhibit the onset of diabetic retinopathy. Cephalotaxine may be administered to a host that is at risk of exhibiting macular degeneration (such as an elderly human) as a prophylactic treatment to prevent or inhibit the onset of macular degeneration.

For the prophylactic treatments above, the cephalotaxine Is administered in amount sufficient to inhibit the onset or progression of the angiogenic disease.

The oral cephalotaxine dosage form is administered to a host in the range of 0-05-5.0 mg/m$^2$. Preferably, the cephalotaxine is administered to a host in the range of 0.1 to 3.0 mg/m$^2$. Also preferably, the cephalotaxine is administered to a host in the range of 0.1- 1.0 mg/m$^2$.

The cephalotaxine may be administered biweekly, weekly, daily, twice daily, or more frequently as required to inhibit angiogenesis or to inhibit the onset or progression of an angiogenic disease.

## B. Leukemia

Oral dosage forms of cephalotaxine can also be used to treat leukemia and pre-leukemia conditions. Leukemias that can be treated with oral administration of cephalotaxine include chronic myelogenous leukemia (CML), acute myelogenous leukemia (AML), acute lymphocytic leukemia (ALL), chronic lymphocytic leukemia (CLL) and acute promyelocytic leukemia (APL).

In addition, cephalotaxine can be used to prevent leukemia by administering an oral dosage form comprising cephalotaxine to a patient with a pre-leukemic syndrome such myelodysplastic syndrome.

The oral cephalotaxine dosage form preferably is administered to a host in the range of 0.05-5.0 mg/m$^2$ Preferably, the cephalotaxine is administered to a host in the range of 0.1 to 3.0 mg/m$^2$. Also preferably, the cephalotaxine is administered to a host in the range of 0.1-1.0 mg/m$^2$.

The cephalotaxine may be orally administered biweekly, weekly, daily, twice daily, or more frequently as required to treat the leukemia or to inhibit the onset or progression leukemia.

## III. Second Active Ingredients

The cephalotaxine oral dosage forms may be administered with other active compounds. Such second active compounds can be administered via oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy. When administered orally, such second active ingredients can be formulated with the cephalotaxine as a unit dosage form or as a separate unit dosage form discussed herein.

Examples of active compounds that may be co-administered with the cephalotaxine composition include, but are not limited to, other antiangiogenic agents such as tyrosine kinase inhibitors, angiostatins, VEGF inhibitors such as Avastin® (bevacizumab), endostatins, combretastatins, 2-methoxy-estradiol, thalidomide, taxanes, antimetabolites such as methotrexate, corticosteroids, colchicine and analogs, antibodies against angiogenic targets, interferon, diabetic regulating agents such as insulin and insulin growth factor inhibitors, non-steroidal anti-inflammatory agents NSAIDS, and anti-arthritics

The oral dosage form may comprise an NSAID. Suitable compounds having NSAID activity include, but are non-limited to, the nonselective COX inhibitors, selective COX-2 inhibitors, selective COX-I inhibitors, and COX- LOX inhibitors, as well as pharmaceutically acceptable salts, isomers, enantiomers, solvates, hydrates, polymorphic crystal forms including the amorphous form, co-crystals, derivatives, prodrugs thereof.

Exemplary NSAIDs include. but are not limited to, celecoxib (Celebrex™); rofecoxib (Vioxx™), etoricoxib (Arcoxia™), meloxicam (Mobic™), valdecoxib, diclofenac (Voltaren™, Cataflam™), etodolac (LodineTM), sulindac (Clinori™), aspirin, alclofenac, fenclofenac, diflunisal (Dolobid™), benorylate, fosfosal, salicylic acid including acetylsalicylic acid, sodium acetylsalicylic acid, calcium acetylsalicylic acid, and sodium salicylate; ibuprofen (Motrin), ketoprofen, carprofen, fenbufen, flurbiprofen, oxaprozin, suprofen, triaprofenic acid, fenoprofen, indaprofen, piroprofen, flufenamic, mefenamic, meclofenamic, niflumic, salsalate, rolmerin, fentiazac, tilomisole, oxyphenbutazone, phenylbutazone, apazone, feprazone, sudoxicam, isoxicam, tenoxicam, piroxicam (Feldene™), indomethacin (Indocin™), nabumetone (Relafen™), naproxen (Naprosyn™), tolmetin, lumiracoxib, parecoxib, licofelone (ML3000), including pharmaceutically acceptable salts, isomers, enantiomers, derivatives, prodrugs, crystal polymorphs, amorphous modifications, co-crystals, solvates, hydrates, and combinations thereof.

The active ingredient may administered to the host before, during or after administration of the cephalotaxine composition. The active ingredient may be mixed with the cephalotaxine prior to administration and the mixture may be administered to the host The active ingredient and the cephalotaxine may be administered separately but simultaneously to the host. The active ingredient may be administered before the cephalotaxine. Preferably, the second active ingredient is administered before the cephalotaxine with the active ingredient still present systemically in the host. The active ingredient may be administered after the cephalotaxine. Preferably, the active ingredient is administered after the cephalotaxine while the cephalotaxine is still present systemically in the host.

## IV. Oral Dosage Forms

The present disclosure provides oral dosage forms. The oral dosage form comprises a cephalotaxine and a pharmaceutically acceptable carrier as described hereinbefore. The oral dosage form can further comprise one or more excipients or dilutants. In many cases, the carrier increases bio-availability of the oral cephalotaxine dosage form as compared to an oral dosage form that does not contain the same carrier.

### A. Pharmaceutically Acceptable Carriers

The pharmaceutically acceptable carriers are selected from proteins, lipids and combinations thereof as described hereinbefore. The cephalotaxine can be combined with the carrier in an appropriate diluant to form a solution or a suspension. Such liquid formulations can be viscous or non-viscous depending on the amount and the carrier used. The liquid formulations preferably contain between 0.1 to 5 mg of cephalotaxine per ml, more preferably between 1 and 3 mg of cephalotaxine per ml.

The liquid formulations can be used as is or can be further formulated into an appropriate capsule or gel capsule. Alternatively, the liquid formulation can be converted into a solid by methods know to those skilled in the art. Such solid formulations can be used as a powder or formulated into granules, capsules, tablets or films any one of which can be made as a time release formulation.

Alternatively, solid cephalotaxine can be combined with solid carrier and used as a powder or formulated into granules, capsules, tablets or films any one of which can be made as a time release formulation.

#### (i) Proteins

Protein that can be used as a carrier, is selected from the group consisting of casein, sodium caseinate, whey protein concentrate, gelatin, soy protein (isolated), brown algae protein, red algae protein, bakers yeast extract and albumin, such as bovine serum albumin or human serum albumin. The concentration is from about 1 mg/ml to 100 mg/ml for a non- viscous liquid formulation and from about 10 mg/ml to 1000 mg/ ml for a viscous liquid formulation. For solid formulations the amount of protein is from about 10 mg/mg of cephalotaxine to 300 mg/mg of cephalotaxine, more preferably from about 20 mg/mg of cephalotaxine to 200 mg/mg of cephalotaxine.

#### (ii) Lipids

Lipid that can be used as a carrier is selected from the group consisting of oleic acid, coconut oil (refined), soybean oil (hydrogenated) and rapeseed oil, aluminum palmitate, dilauryl thiodipropionate, enzyme-modified lecithin, calcium stearate, enzyme-modified fats, glyceryl palmitostereate, lecithin, mono- and diglycerides and vegetable oil. The concentration is from about 1 mg/ml to 100 mg/ ml for a non-viscous liquid formulation and from about 10 mg/ml to 100 mg/ ml for a viscous liquid formulation. For solid formulations the amount of lipid is from about 10 mg/mg of cephalotaxine to 300 mg/mg of cephalotaxine, more preferably from about 20 mg/mg of cephalotaxine to 200 mg/mg of cephalotaxine.

### (iii) Carrier combinations

Two or more of the protein and lipid carriers can be combined with cephalotaxine to make an oral dosage form. This can include two or more different proteins or lipids or a combination of two or more carriers selected from the group consisting of protein or lipid.

## B. Excipients

The oral dosage forms comprising cephalotaxine and carrier can also include pharmaceutically acceptable excipients, some of which may also fall into the category of carriers as set forth above. If an excipient is a protein or lipid and increases bio-availability, such excipients are considered to be carriers as set forth above.

Excipients include, among others, binding agents, fillers, lubricants, disintegrants, emulsifiers, wetting agents, buffers, plasticizers, diluents, coatings, e.g. enteric coatings, pigments or coloring agents, flow agents, glidants, subcoating materials. Common binding agents include, among others, starch, pregelatinized starch, polyvinylpyrrolidone, methylcellulose, hydroxypropylcellulose, hydroxypropylmethylcellulose and PVP (e.g. Povidone K 29-32). Fillers include, among others, lactose, microcrystalline cellulose, mannitol and calcium hydrogen phosphate. Lubricants include, among others, polyethylene glycol (for example PEG 6000), castor oil, hydrogenated castor oil, magnesium stearate, sodium stearyl fumarate, stearic acid and talc. Disintegrants for controlling dissolution and dispersion of the oral formulation include, among others, modified starch, sodium starch glycolate, crospovidone or croscarmellose sodium. Emulsifying and wetting agents include various surfactants both ionic and nonionic and natural or synthetic origin, for example, phospholipids, polysorbates, lecithin, oxypropylene polymers, polyethylene glycols, tweens, pluronics and sodium lauryl sulfate. By way of example, pharma grade sugar can be used in the core for omeprazole pellets or granules; dibasic sodium phosphate as a buffer and diluent; calcium carbonate as an alkaline material and dusting agents or powder for subcoating; sodium lauryl sulfate as a surfactant; hypromellose as a binder or coating; methacrylic acid copolymer as an enteric coating; diethyl phthalate as a plasticizer; talc as a lubricant/glidant; titanium dioxide as a pigment or coloring agent; starch as a disintegrant or core; and colloidal silica as a flow agent or glidant.

In addition to the acceptable excipients described above, various additives may be included in the oral dosage forms of the invention. These include, but are not limited to, pharmaceutically acceptable flavoring agents, sweeteners, stabilizing agents, preservatives, anti-microbial agents, flow aids, coloring agents, antioxidants, wetting agents, surfactants, emulsifiers, efflux inhibitors and other excipients know to one skilled in the art.

Sweetening or flavoring agents, when present, are preferably in an amount of from about 0.1 to about 80% by weight based on the total weight of the oral dosage form. Suitable sweetening or flavoring agents are well known in the art. Exemplary sweetening agents include, but are not limited to, dextrose, polydextrose, mannitol, saccharine, sorbitol, sucrose, aspartame, acesulfame K, or xylitol.

The oral dosage forms optionally contain pharmaceutically acceptable coloring agents, water-soluble dyes or pigments. Typical coloring agents include, among others, synthetic iron oxides, e.g., FD&C Red, and FD&C Blue. The oral dosage forms optionally contain pharmaceutically acceptable opacifiers, including but not limited to talc.

A variety of oral dosage forms are disclosed herein and will be apparent to the skilled artisan. Representative formulation technology is taught in. *inter alia.* Remington: The Science and Practice of Pharmacy, 21st Ed., Mack Publishing Co., Easton, PA (2006) and Handbook of Pharmaceutical Excipients, 3rd Ed, Kibbe, A.H. ed., Washington DC, American Pharmaceutical Association (2000), or in Pharmaceutical Dosage Forms - Tablets, Lieberman, Herbert A., Lachman, Leon, et al., eds., Marcel Dekker Inc. (1998). Exemplary oral dosage forms of the present invention can include solids, tablets, capsules, powders, granules, solutions, suspensions, films and other formulations known in the art. Each of the oral dosage forms can optionally comprise an enteric coating. The oral dosage forms can be provided in a single unit or in multiple units. The oral dosage forms may be provided in packets, bottles, blisters, sachets, and other types of containers, and where appropriate, accompanied by a desiccant to provide moisture protection. The oral dosage form may also be provided in a device for providing a measured dose.

In a specific embodiment, the oral dosage form comprises between about 0.1 mg and 5 mg of cephalotaxine. In another exemplary embodiment, the oral dosage form comprises about 1 mg to 3 mg of cephalotaxine. In another exemplary embodiment, the oral dosage form is a capsule or a tablet..

Combination pharmaceutical compositions are known in the art see for example, U.S. Patent Nos. 6,926,907; 6,599,529; 6,365,184; 6,869,615; 6,184,220; 6,284,269, 6,682,747; 6,613,354 and 6,740,340. The cephalotaxine, carrier, second active ingredient and optional excipients can be compressed into a tablet. In some embodiments, the tablet can comprise one or more enteric coatings. A variety of tablet formulations with oral dosage forms, combination pharmaceuticals and/or enteric coatings are known in the art, see for example U.S. Patent Nos. 6,613,354 and 6,740,340.

### (i) Granules

In some embodiments, one or more of the active ingredients in the oral dosage forms described herein are located on and/or embedded within granules, pellets, or beads. These granules can contain a selected percentage of one or more active ingredients, with the remainder of its mass consisting of inactive ingredients. Examples of these inactive ingredients include, but are not limited to, sugar, calcium carbonate, sodium bicarbonate, sodium phosphate dibasic, methacrylic acid copolymer, cellulose acid phthalate, hydroxymethylpropylcellulose phthalate, hydroxypropylcellulose and hypromellose.

The percentage of the weight of the active ingredient which constitutes the weight of the granule is known as the drug loading level. The drug loading level for an active ingredient in an oral dosage form is an average. Some of the granules may contain little or no active ingredient, while others may contain greater amounts than the percentage stated. To the extent that a physically smaller oral dosage form is desired, the drug loading level will need to be increased. Higher drug loading levels allow the physical size of the oral dosage form to be decreased.

In another exemplary embodiment, the cephalotaxine and carrier are present in and/or on granules. In yet another exemplary embodiment, the cephalotaxine and carrier are present in and/or on granules, and the granules have an enteric coat. In an exemplary embodiment, the drug loading level of cephalotaxine in and/or on a granule in an oral dosage form described herein is from about 1% to about 50%. In another exemplary embodiment, the drug loading level of cephalotaxine is from about 5% to about 25%. In yet another exemplary embodiment, the drug loading level of the cephalotaxine is from about 6% to about 15%. In yet another exemplary embodiment, the drug loading level of the cephalotaxine is from about 7% to about 13%. In yet another exemplary embodiment, the drug loading level of the cephalotaxine is from about 8% to about 12%.

### (ii) Enteric coatings

In some embodiments, one or more active ingredients in the oral dosage forms described herein are enterically coated. In other embodiments, one or more units of the oral dosage forms described herein are enterically coated. In another exemplary embodiment, essentially all of the cephalotaxine and carrier is enterically coated. Generally, the enteric material is insoluble in acid environments, such as the stomach, but is soluble in near-neutral environments such as the small intestine. Because of the enteric properties, the coated material can pass through the stomach essentially undissolved and can be released in the lower part of the intestinal tract. In some embodiments, the enteric coating dissolves at a pH of between 5 and 7.5.

In some embodiments, the oral dosage form comprises granules that are enterically coated prior to being incorporated into the oral dosage form. In some embodiments where a second active ingredient is used, the cephalotaxine is enterically coated and the second active ingredient is also enterically coated. In other embodiments, the cephalotaxine is enterically coated and the second active ingredient is not enterically coated. In other embodiments, an enteric coat covers both the cephalotaxine and the second active ingredient, and any other components of the oral dosage form such as the carrier and optional excipient. In some embodiments, the cephalotaxine is in the form of enteric coated multiparticulate units and the second active ingredient is in the form of granules or alternatively in the form of modified release formulated units such as enteric coating layered units or units layered with a controlled release layer Examples of multiparticulate units are pellets, granules, or beads.

Various enteric materials are known in the art, a number of which are commercially available. Exemplary enteric coatings of use in the present invention can be any enteric material known to those skilled in the art, see for example, U.S. Patent Nos. 6,855,702 and 6,605,300. The enteric materials usually comprise a polymer with enteric properties. Suitable non-limiting examples include methacrylic acid copolymers such as methacrylic acid/methyl methacrylate copolymers, methacrylic acid/ethyl acrylate copolymers, methacrylic acid/methyl acrylate/methyl methacrylate copolymers, shellac, hydroxypropyl methylcellulose phthalate, hydroxypropyl methyl cellulose acetate-succinates, hydroxypropylmethylcellulose trimellitate, cellulose acetate-phthalates, polyvinyl acetate phthalate or a mixture of these components, or other suitable enteric materials.

In some embodiments, enteric coating layer(s) are applied using standard coating techniques. The enteric coating is applied using a variety of methods known in the art, such as spraying or layering see for example U.S. Patent Nos. 4,287,221 and 6,605,300. The thickness of the enteric coating is designed based on the nature of the coating material and the desired lag time or delay in release of the oral dosage form ingredients. The enteric coating(s) may be applied to the outside surface of an oral dosage form, or a coating, using a suitable coating technique. The enteric coating layer material may be dispersed or dissolved in either water or in a suitable organic solvent for coating.

In some embodiments the enteric coating contains an effective amount of a pharmaceutically acceptable plasticizer to obtain the desired mechanical properties, such as flexibility of the enteric coating layers. Such plasticizers are, for example and without limitation, triacetin, citric acid esters, phthalic acid esters, dibutyl sebacate, cetyl alcohol, diethyl phthalate, triethyl citrate, polyethylene glycols, polysorbates or other plasticizers. The amount of plasticizer is optimized

for the particular situation. The amount of plasticizer is preferably above 10% by weight of the enteric coating polymer(s), preferably 15-50%, and more preferably 20-50%. Additives such as dispersants, colorants, pigments, anti-tacking agents may also be included into the enteric coating layer(s). Other compounds may be added to increase film thickness or opacity and to decrease diffusion of acidic gastric juices into the dosage form.

As will be appreciated by one skilled in the art, overcoating may be applied to the enteric coated oral dosage form, for example, as a protective layer, flavor, and the like. Suitable overcoating materials include, but are not limited to, sugar, polyethylene glycol, polyvinylpyrrolidone, polyvinyl alcohol, polyvinyl acetate, hydroxypropyl cellulose, methylcellulose, ethylcellulose, hydroxypropyl methyl cellulose, carboxymethylcellulose sodium and the like. Additives such as plasticizers, colorants, pigments, fillers, anti-tacking and anti-static agents, such as for instance magnesium stearate, titanium dioxide, talc and other additives may also be included in the over-coating layer(s).

**(iii) Capsules**

In an exemplary embodiment, the oral dosage form described herein can be provided as orally administrable capsules, e.g. hard or soft gelatin capsules, or other encapsulated dosage forms such as HPMC capsules, known in the art. The capsule wall can include any of the various materials conventionally used in the pharmaceutical industry, including, by way of example and not limitation, gelatin, carrageenins, polysaccharide (*e.g.*, agar, hydroxypropyl methylcellulose, hydroxyethylcellulose, pectin, starch *etc.* or mixtures thereof). Suitable hard gelatin capsules are supplied by Capsugel. Suitable HPMC capsules are supplied by Shinogi.

The orally administrable capsule can comprise substrates, suspensions, or an aqueous solution of the cephalotaxine or cephalotaxine analog and optionally a second active ingredient. For example, hard gelatin capsules can be filled with powders, warm solutions or suspensions of the cephalotaxine or cephalotaxine analog in waxy and/or lipid based formulations that solidify in the capsule when cooled to room temperature. Soft gel capsules can be filled with solutions or suspensions of the cephalotaxine or cephalotaxine analog and optionally the second active ingredient in oil and/or lipid and/or solvents such as PEG or propylene glycol.

The cephalotaxine or cephalotaxine analog and optionally the second active ingredient can be dry mixed and filled into a capsule. The capsule can include a plasticizer, such as glycerin, triacetin, sorbitol, polyethylene glycol, propylene glycol, citrate, and phthalate, to impart form and flexibility where desired.

In some embodiments, the orally administrable capsule includes an enteric coating. In some embodiments, substrates containing the cephalotaxine or cephalotaxine analog and optionally the second active ingredient, such as granules, are enterically coated prior to being filed in the capsule. In some embodiments, the cephalotaxine or cephalotaxine analog is in the form of enteric coated multiparticulate units and the second active ingredient is in the form of granules or alternatively in the form of modified release formulated units such as enteric coating layered units or units layered with a controlled release layer.

In a specific embodiment, the capsule comprises about 0.1 to 5 mg, preferably about 0.5 to 2 mg, of cephalotaxine

**(iv) Conventional tablets**

The oral dosage forms described herein may be in the form of a conventional compressed tablet. See, Remington's Pharmaceutical Sciences, Mack Publishing. In an exemplary embodiment, the cephalotaxine and carrier may be intimately mixed with each other and compressed into a conventional tablet. In those embodiments using a second active ingredient, the cephalotaxine and second active ingredient may be intimately mixed with each other and compressed into a conventional tablet.

The tableted dosage form can include an enteric coated cephalotaxine with a second active ingredient optionally constituting the rest of the active ingredients of the compressed tablet.

Alternatively, the cephalotaxine and optionally the second active ingredient may be intimately mixed with each other and compressed into a conventional tablet and the entire tablet enterically coated. In this configuration, the tablet contains cephalotaxine and the second active ingredient in the required doses along with appropriate excipients, and optionally agents to aid dissolution, lubricants, fillers, *etc.* Here, the entire tablet may be enterically coated.

**(v) Multilayer tablets**

Another suitable dosage form is a multilayer tablet. In the multilayer tablet, the first component may be compressed into one layer, with the second component being subsequently added as a second layer of the multilayer tablet. Optionally, one or more subcoats or barrier coats may be added prior to the second layer or prior to adding an enteric coating.

In a specific embodiment, the multilayer tablet comprises one portion containing cephalotaxine, optionally combined with appropriate excipients, agents to aid dissolution, lubricants, fillers, *etc.* The second portion of the tablet

comprises the second active ingredient, optionally combined with other excipients, dissolution agents, lubricants, fillers, *etc.* In some embodiments, the cephalotaxine or the second active ingredient can be enterically coated. Alternatively, a layer of the multilayer tablet, or the entire tablet can be enterically coated.

In some embodiments, the multilayer tablet comprises the cephalotaxine in the core of the tablet and the second active ingredient covers the core. In some embodiments, second active ingredient is in the core of the tablet and the cephalotaxine covers the core. In some embodiments, the cephalotaxine can be enterically coated granules. Alternatively, in some embodiments the multilayer tablet comprises the second active ingredient in the core of the tablet and the cephalotaxine covers the core, wherein the entire tablet is enterically coated.

**(vi) Controlled release**

In some embodiments, the oral dosage forms described herein provide controlled release of one or more active ingredients using one or more controlled release agents. A variety of controlled release pharmaceutical compositions are known in the art, see for example U.S. Patent Nos. 6,861,072 and 6,905,708. The term "controlled release" is intended to mean the release of one or more active ingredients at a pre-selected or desired rate. This rate will vary depending upon the application. Desirable rates include fast or immediate release profiles as well as delayed release, sustained release or sequential release. Combinations of release patterns, such as initial release followed by lower levels of sustained release of active ingredient are specifically contemplated.

**(vii) Effervescents**

The active agents may further be included in an effervescent dosage form. One or more effervescent agents can be used as disintegrants and/or to enhance organoleptic properties of compositions of the invention. When present in compositions of the invention to promote dosage form disintegration, one or more effervescent agents are preferably present in a total amount of about 30% to about 75%, and preferably about 45% to about 70%, for example about 60%, by weight of the composition.

In some embodiments, an effervescent agent, present in a dosage form in an amount less than that effective to promote disintegration of the dosage form, provides improved dispersion of the actives in an aqueous medium. Without being bound by theory, the effervescent agent can be effective to accelerate dispersion of one or more active ingredients from the dosage form in the gastrointestinal tract, thereby further enhancing absorption and rapid onset of therapeutic effect. When present in a dosage form of the invention to promote intragastrointestinal dispersion but not to enhance disintegration, an effervescent agent is preferably present in an amount of about 1% to about 20%, more preferably about 2.5% to about 15%, and still more preferably about 5% to about 10%, by weight of the composition.

An effervescent agent is an agent comprising one or more compounds which, acting together or individually, evolve a gas on contact with water. The gas evolved is generally oxygen or carbon dioxide. Preferred effervescent agents comprise an acid and a base that react in the presence of water to generate carbon dioxide gas. Preferably, the base comprises an alkali metal or alkaline earth metal carbonate or bicarbonate and the acid comprises an aliphatic carboxylic acid.

Non-limiting examples of suitable bases as components of effervescent agents useful in the invention include carbonate salts (*e.g.*, calcium carbonate), bicarbonate salts (*e.g.*, sodium bicarbonate), sesquicarbonate salts, and mixtures thereof.

Non-limiting examples of suitable acids as components of effervescent agents useful in the invention include citric acid, tartaric acid, malic acid, fumaric acid, adipic acid, succinic acid, acid anhydrides of such acids, acid salts of such acids, and mixtures thereof.

Other components of the tablets may include but are not limited to microcrystalline cellulose, lactose, mannitol, croscarmellose sodium and magnesium stearate.

**(viii) Orally administrable tablets**

Orally administrable tablets can be used, either as a single unit or multiunit oral dosage form or as part of a multiunit oral dosage form. In an exemplary embodiment, the single unit orally administrable tablet can comprise an antiplatelet agent, and an acid inhibitor see for example U.S. Patent No. 6,723,348; 6,692,771; 6,365,182; 6,221,392; 6,899,899; and 7,008,640. In some embodiments, the orally administrable tablet comprises cephalotaxine and optionally a carrier. Pharmaceutically acceptable excipients can also be used and include, but are not limited to, surfactants and agents, such as sodium bicarbonate, to improve the dissolution or absorption.

**(ix) Chewable tablets**

In an exemplary embodiment, the oral dosage form described herein can be in a chewable tablet. Chewable tablets usually contain large amounts of pleasant-tasting substances such as mannitol in the formulation, and are known in the art, see for example U.S. Patent Nos. 7,014,862 and 7,008,640. In a specific embodiment, the chewable tablet comprises cephalotaxine and optionally a carrier. The chewable tablet may also include one or more excipients.

**(x) Orally administrable films**

In an exemplary embodiment, the oral dosage form described herein can be in the form of an orally administrable film. It is specifically contemplated that the amount of active ingredient(s) in the film is dependent on the type of film, thickness and surface area of film.

It is contemplated that the orally administrable films described herein can comprise a single film layer or multiple film layers. For example, it may be desirable to form an orally administrable film comprising a cephalotaxine or a multilayer film comprising a first film containing cephalotaxine and opionally a carrier and/or excipient and a second film comprising a second active ingredient which may be layered onto the first film. One or more of the films may impart modified release characteristics to the formulation.

In some embodiments, the orally administrable film comprises cephalotaxine and a second active ingredient

Orally administrable films and methods for making such films are well known in the art. See for example: U.S. Patent Nos. 4,136,145; 4,713,243; 5,166,233; 5,700,478; 5,800,832, 5,948,430; 6,419,903, 6,177,096; 6,284,264; 6,596,298; 6,656,493; 6,709,671; 6,824,829; 6,923,981, and United States Patent Application Publication Nos.: US 2001/0046511; US 2001/0022964; US 2002/0131990; US 2003/0107149; US 2004/0151756, US 2004/0241242; US 2004/0247649; US 2004/0258896; US 2005/0184427; US 2005/0196358; US 2005/0075432 and US 2005/0037055.

The orally administrable film can be prepared as described in U.S. Patent No. 6,709,671, for example. Polyalcohol, surfactants, plasticizers, and possible other ingredients except the water-soluble or water-dispersible polymers are dissolved in a sufficient amount of a solvent which is compatible with them. Examples of compatible solvents include water, alcohols or mixtures thereof. After a clear solution is formed, the water-dispersible polymer or mixture of water-dispersible polymers is slowly added with stirring, and heating if necessary, until a clear and homogeneous solution is formed, followed by the addition of cephalotaxine alone or in combination with a carrier, excipient and/or second active ingredient and flavors. The solution is coated onto a suitable carrier material and dried to form a film.

In some embodiments, the orally administrable film can be prepared as described in U.S. Patent Appl. No. 2005/0184427. Briefly, the desired components are combined to form a multi-component matrix, including the polymer, water, and cephalotaxine and optionally a carrier, excipient and/or the second active ingredient as desired, and the combination is formed into a sheet or film, by any method known in the art such as extrusion, coating, spreading, casting or drawing the multi-component matrix. If a multi-layered film is desired, this may be formed by co-extruding more than one combination of components which may be of the same or different composition. A multi-layered film may also be formed by coating, spreading, or casting a combination of components onto an already formed film layer.

As described above, the cephalotaxine and optionally the second active ingredient can be mixed with the film forming solution to form the desired orally administrable film. The active ingredients can be uniformly dispersed in the film forming solution in the form of insoluble solid particles together and/or as soluble active ingredients. In some embodiments, granules containing the cephalotaxine and optionally the second active ingredient are added to the film forming solution.

In some embodiments, cephalotaxine may be added to the film forming polymer solution in the form of granules together with an enteric coated granules containing the second active ingredient. In some embodiments, the second active ingredient may be added to the film forming polymer solution in the form of granules together with the enteric coated granules containing cephalotaxine.

In some embodiments, the cephalotaxine and optionally second active ingredient may be incorporated into the film-forming mixture in liquid form, such as in solution or suspension rather than as film coated solid particles.

The orally administrable films generally comprise one or more polymers as well as fillers as desired. Film-forming polymers are well known in the art. See for example, U.S. Patent Appl. No. 11/092217. Generally, the polymer can be water soluble, water insoluble, water swellable or a combination thereof. In some embodiments the polymer can include cellulose or a cellulose derivative. Suitable non-limiting examples of water soluble polymers include carboxymethyl cellulose, hydroxypropylmethyl cellulose, hydroxyethyl cellulose, hydroxypropyl cellulose, polyvinyl pyrrolidone, polyvinyl alcohol, pullulansodium aginate, polyethylene glycol, acacia gum, arabic gum, xanthan gum, tragancanth gum, guar gum, polyacrylic acid, methylmethacrylate copolymer, carboxyvinyl copolymers, starch, and combinations thereof. Suitable non-limiting examples of water insoluble polymers include cellulose acetate, hydroxypropyl ethyl cellulose, hydroxypropyl methyl cellulose, phthalateethyl cellulose, phthalate and combinations thereof.

The concentration of enteric coated active ingredient(s) in the orally administrable films should be suitable for

therapeutic benefit without causing adverse feeling, such as grittiness, in the mouth. The amount of enteric coated cephalotaxine and optionally second active ingredient in the orally administrable films depends on the kind of active and is usually between about 0.01 and about 20% to as high as 30% (w/w), and it can be higher if necessary to achieve the desired effect.

In some embodiments, orally administrable films provide controlled release of one or more active ingredients using one or more controlled release agents. In some embodiments, controlled release can be achieved by providing a substantially water insoluble film that incorporates one or more active ingredients that will be released from the film over time. In some embodiments, a variety of different water soluble or insoluble polymers can be used and optionally include biodegradable polymers in combination.

In some embodiments, one or more active ingredients employed in the present invention can be incorporated into the film in a controlled release form. For example, active ingredients can be coated with polymers such as ethyl cellulose or polymethacrylate.

Additional components can be incorporated into the cephalotaxine containing films, including, without limitation, colorants, flavors, fragrances, mouthwash components, preservatives, sweetening agents, vitamins, antioxidants and combinations thereof. Additional components can include, without limitation, surfactants and plasticizers for compartmentalizing the components within the mixture; polyalcohols; and thermo-setting gels such as pectin, carageenins, and gelatin, which can help maintain the dispersion of components. Citric acid, or other suitable agent, can be added to stimulate saliva production and facilitate rapid dissolution of the film in the oral cavity, and/or provide an acidic environment for an enteric coated proton pump inhibitor.

In some embodiments, the dissolving film can be adhered to the oral cavity thereby releasing the active ingredients of the oral dosage form. In some embodiments, the dissolving film can be adhered to the oral cavity thereby releasing some of the active ingredients locally in the oral cavity

Optionally, the orally administrable film formulation may contain a combination of plasticizers, surfactants, colorants, sweetening agents, flavors, flavor enhancers, and/or other excipients commonly used to modify the taste of formulations intended for application to the oral cavity.

The orally administrable films provided herein can accommodate a wide range of amounts of the active ingredients. As understood by one skilled in the art, the amount of active ingredients incorporated into the film depends in part on the type of film, polymer, surface area, and thickness of the film. In some embodiments, the amount of active ingredient(s) to film is between about 0.01 and about 50% (w/w), but it can be higher if necessary to achieve the desired effect.

**(xi) Active Ingredients in separate units**

In some embodiments, a package can comprise a unit of a therapeutically effective amount of cephalotaxine and a separate unit of a therapeutically effective amount of a second active agent. In some embodiments, the oral dosage form can be provided in packaging in which one or more cephalotaxine and a second active ingredient are provided in separate units in the same oral dosage form or package or container, for co-administration. Suitably, the units for each of the cephalotaxine and second active ingredient can be tablets, capsules, films, powders, granules, solutions, solids, suspensions and or other acceptable oral dosage forms. For example, one of the units can contain the cephalotaxine but not the second active ingredient and another of the units in the packaging can contain the second active ingredient but not the cephalotaxine. These combinations may be provided, for example, in packaging, such as kits, blister packs, packets or bottles shrink-wrapped together in which more than one dosage form of the various components are provided in the same dispensing unit for co-administration.

In some embodiments a kit is provided in which one or more sheets of blister packs comprising a dosage form of cephalotaxine and one or more sheets of blister packs comprising dosage forms of a second active ingredient. In some embodiments, the kit comprises a plurality of sheets of blister packs, wherein each sheet comprises at least one blister pack comprising the dosage form of one or more cephalotaxine and at least one blister pack comprising the dosage form of a second active ingredient. The dosage form for each of the cephalotaxine and second active ingredient is selected from the group consisting of tablets, capsules, films, powders, granules, solutions, solids, suspensions and another acceptable oral dosage forms.

The oral dosage forms can be packaged in sealed, air and moisture resistant packages to protect the active ingredients from exposure to the environment and from oxidation, hydrolysis, volatilization resulting from interaction with the environment. The packaged oral dosage forms can contain a fill supply of the medication typically prescribed for the intended therapy. A series of unit doses can be packaged together in accordance with the prescribed regimen or treatment, e.g., a 3-90 day supply, depending on the particular therapy.

A number of benefits are derived from the oral dosage forms provided herein. For example, the orally administrable tablets, chewable tablets and oral film strip formulations can be administered without water. These methods of drug administration, without the need for water, are also particularly well suited for a mobile society. The oral dosage

forms provided herein can be particularly appealing to subjects with difficulty in swallowing pharmaceuticals, such as children, elderly, and also in veterinary practice. For example, dosage forms, including tablets and films, placed in the sublingual area are suitable for non-enteric coated acid inhibitors as absorption from a sublingual dosage form is generally fast and avoids first pass metabolism.

In addition, the oral dosage forms provided herein provide for accurate dosage amounts. For example, in the oral film strip formulations the dosage amount can be determined by the size of the film and concentration of the active ingredient(s) in the original polymer/water or polymer/solvent combination.

## V. Methods of Use

Provided herein are methods of preventing or treating a cephalotaxine sensitive disease or syndrome in a subject comprising orally administering, either as a single unit or as multiple units, a therapeutically effective amount of cephalotaxine.

Also disclosed are methods of preventing or treating a cephalotaxine sensitive disease or syndrome in a subject comprising orally administering, either as a single unit or as multiple units, a therapeutically effective amount of cephalotaxine and co-administrating a second active ingredient.

The term "co-administration" refers to the administration of one or more cephalotaxines and optionally a second active ingredient, at approximately the same time or in close sequence so that their effects run approximately concurrently or substantially overlap. Although no specific time is required, co-administration can be within 60, 30, 15, 10, 5, or 1 minute or less.

Provided herein are methods of inhibiting a cephalotaxine sensitive disease or syndrome in a subject who is not otherwise in need of such treatment, comprising administering an oral dosage form which includes a therapeutically effective amount of cephalotaxine and optionally a second active ingredient.

The oral formulations and methods described herein can be administered to any subject in need of therapy including, without limitation, humans, including patients, companion animals, including, but not limited to dogs, cats, ferrets, and birds, food-source animals, including, but not limited to cows, pigs, and sheep, and zoo animals, such as monkeys and other primates, and other similar animal species

The present disclosure includes a method of preventing or reducing the severity, duration, and/or symptoms of a cephalotaxine sensitive disease, disorder or syndrome comprising orally administering an oral dosage form of cephalotaxine alone or in combination with a second active ingredient to a patient.

The present disclosure includes a method of preventing or reducing the severity, duration, and/or symptoms of a cephalotaxine sensitive disease, disorder or syndrome comprising orally co -administering an oral dosage form of cephalotaxine or cephalotaxine analog and a second active ingredient to a patient.

The foregoing descriptions of specific embodiments of the present invention have been presented for purposes of illustration and description. They are not intended to be exhaustive or to limit the invention to the precise forms disclosed, and obviously many modifications and variations are possible in light of the above teaching. The embodiments were chosen and described in order to best explain the principles of the invention and its practical application, to thereby enable others skilled in the art to best utilize the invention and various embodiments with various modifications as are suited to the particular use contemplated. It is intended that the scope of the invention be defined by the Claims appended hereto and their equivalents.

## EXAMPLES

Aspects of the present teachings may further be understood in light of the following examples, which should not be construed as limiting the scope of the present teachings in any way.

### EXAMPLE 1

To evaluate the potential for the development of an oral formulation of homoharringtonine, a pharmacokinetic study was performed in mice. Homoharringtonine was given, at 2 doses, by either oral or subcutaneous routes to male CD-I mice. After a single administration, blood was collected from the tail vein and analyzed for homoharringtonine content. The animal dosing was performed by Murigenics (Berkeley, CA) and the analysis of sera was performed by PHARMout (Sunnyvale, CA). The bioavailability of homoharringtonine after oral delivery was 76-77%, depending on dose given, as compared to subcutaneous delivery. This bioavailability is considered very good.

Homoharringtonine was manufactured by Stragen for ChemGenex Pharmaceuticals. 5 mg of lyophilized homoharringtonine was provided in a glass vial and was stable at room temperature. The vehicle was 0,9% sodium chloride supplied with the test agent in a separate vial. 27 male CD-I mice, 8-10 weeks old (Charles River Laboratories) were used for this study. 24 mice were used for dosing, and 3 mice were used as naïve controls.

On the day of the study, 5 ml of saline were added to the 5 mg vial to make a stock of 1 mg/ml HHT. The amount administered for each mouse was based on weight, using a dose volume of 4 ml/kg. The dosing administration was performed according to Table 1.

**Table 1**

| Group | Number of Animals | Test Article | Dose Levels (mg/kg) | Route |
|---|---|---|---|---|
| 1 | 6 | HHT | 0.41 | p.o. |
| 2 | 6 | HHT | 0.41 | s.c. |
| 3 | 6 | HHT | 1.6 | p.o. |
| 4 | 6 | HHT | 1.6 | s.c. |

Test subjects were dosed once. The enzyme blocker was provided in two separate tubes (A & B). Immediately prior to blood collection, 20 μL each of A and B were aliquoted into a small tube (to yield 40 μL of enzyme inhibitor).

Blood from 3 naïve mice was collected for a 0 timepoint. Approximately 50 μL of whole blood per animal (n=3 mice per time point) was collected into heparinized capillary tubes. Heparanized blood was transferred into tubes containing 40 μL of enzyme inhibitor. Samples were clearly marked by PK timepoint and group. Blood was stored on ice until centrifugation. There were 6 mice treated at each dose, but blood was pooled from 3 mice per time point so that each mouse had blood collected at every other timepoint. There were the following timepoints: 0, 15 min, 30min, 2hr, 4hr and 8hr after the administration of homoharringtonine. There were three samples collected for the T=0 from naive mice. There were 5 samples collected for each dose, corresponding to the timepoints, 15 min, 30min, 2hr, 4hr and 8hr after the administration of homoharrigntonine. There were 2 doses for each route, and 2 routes of administration, so there were a total of 20 samples collected from treated mice. Plasma tubes were spun to prepare plasma, frozen to -80°C and shipped on dry ice to PHARMout for bioanalytical work up. All animals were observed for 7 days after the last blood collection time.

Animals were observed twice daily (in the morning and in the evening) for mortality and morbidity beginning at the time of arrival from the vendor until the scheduled sacrifice 7 days after the last blood collection. Prior to dosing, each animal was removed from its cage and observed for signs of poor health and abnormal behavior. All animals were weighed prior to dosing. In addition, each animal was observed for adverse events, such as convulsions or death for approximately 10 to 20 minutes following the administration. No mortalities or signs of morbidity were observed through the duration of the experiment.

No unscheduled sacrifices or deaths were recorded. Animals were sacrificed via $CO_2$ 7 days after the completion of blood draws.

Plasma samples were analyzed at PHARMout for HHT content and the results are listed in Table 2 displayed in Figure 3 and summarized in Table 3. These data were analyzed using the WINNONLIN NCA model. At the low dose of 410 μg/kg, oral dosing resulted in 76% of the drug being detected in plasma when compared with subcutaneous dosing. Similarly, the high dose of 1600 μg/kg resulted in 77% oral bioavailability based on the subcutaneous dose. The $C_{max}$ of the oral doses was 4 and 30 ng/mL for the 410 and 1600 μg/kg oral doses, respectively. In contrast, the subcutaneous doses gave $C_{max}$ doses of 20 and 54 ng/mL. A lower $C_{max}$ may result in less toxicity, indicating that oral formulations of cephalotaxines may have safety advantages over subcutaneous formulations. Additionally, the Tmax and T1/2 were higher for the oral doses than for the subcutaneous doses, indicating that therapeutic drug levels may be reached for a longer period after oral dosing as compared to subcutaneous dosing. These results strongly suggest that the development of an oral dosage form of homoharringtonine could not only improve patient convenience and compliance, but may improve therapeutic outcome and reduce toxicity.

**Table 2**

| **Concentrations (ng/mL) of HHT in Mouse Sodium Heparin Plasma Samples** | | |
|---|---|---|
| **Group** | **Time Point (min)** | **Concentration (ng/mL)** |
| Control | - | BQL |
| 1 | 15 | 3.54 |
| 1 | 30 | 3.59 |
| 1 | 60 | 3.85 |

(continued)

| Group | Time Point (min) | Concentration (ng/mL) |
|---|---|---|
| 1 | 120 | 4.29 |
| 1 | 240 | 1.81 |
| 1 | 480 | 1.65 |
| | | |
| 2 | 15 | 20.4 |
| 2 | 30 | 11.2 |
| 2 | 60 | 6.20 |
| 2 | 120 | 3.59 |
| 2 | 240 | 2.44 |
| 2 | 480 | BQL |
| | | |
| 3 | 15 | 6.58 |
| 3 | 30 | 8.87 |
| 3 | 60 | 29.9 |
| 3 | 120 | 9.18 |
| 3 | 240 | 5.52 |
| 3 | 480 | 4.19 |
| | | |
| 4 | 15 | 54.4 |
| 4 | 30 | 15.4 |
| 4 | 60 | 35.3 |
| 4 | 120 | 6.38 |
| 4 | 240 | 7.72 |
| 4 | 480 | 3.10 |
| BQL: Below the quantification limit (1.00 ng/mL) | | |

**Table 3**

| **HHT Pharmacokinetics- PO vs. SC** | | | | | | | | |
|---|---|---|---|---|---|---|---|---|
| Route | Dose ug/kg | Dose mg/m2 | Cmax ng/mL | Tmax | t-1/2 | AUC ng/mL*min | BA po/sc% | Comments |
| po | 410 | 1.2 | 4 | 120 | 297 | 1217 | 76% | Very good BA |
| sc | 410 | 1.2 | 20 | 15 | 215 | 1599 | | |
| po | 1600 | 4.8 | 30 | 60 | 341 | 3966 | 77% | Very good BA |
| sc | 1600 | 4.8 | 54 | 15 | 306 | 5087 | | |

EXAMPLE 2

C3H mice were inoculated subcutaneously in the flank with 2 x $10^5$ radiation-induced fibrosarcoma cells (RIF-1) to produce experimental tumors. When tumors reached ~100 $mm^3$, test agents (100 $\mu L$) were administered orally (PO) or intraperitoneally (IP). Four mice were used in each treatment group. Tumors were measured 3 times a week

using Vernier calipers, and tumor volume (V) was calculated according to the formula:

$$V = \frac{\pi}{6} x D_1 x D_2 x D_3$$

Where $D_{1-3}$ are perpendicular diameters measured in millimeters (mm). Tumor volume quadrupling time was defined as the time (days) for treated and untreated tumors to grow to four times (4x) their initial treatment volume. Tumor growth delay ratio (T/C) was defined as the ratio of 4x growth time of treated (T) and untreated control (C) tumors.

**Delivery Systems**

| **VIscous** | **Non-viscous** |
|---|---|
| Protein Based | Protein Based |
| PDV1: 10% gelatin | PDV2: bovine serum albumin |
| Lipid Based | Saline Based |
| LDV1: partially hydrogenated vegetable oil | SDV1; saline |
| LDV2: stearyl alcohol | |
| Carbohydrate Based (not claimed) | |
| CDV1: honey | |
| CDV2: hydroxypropylcellulose | |
| Combination | |
| LDV1:PDV1 (1:1) | |

Table 4 shows results in the treatment of RIF-I tumors with non-viscous oral homoharringtonine formulations. The results in Table 4 show that the efficacy of HHT in the protein delivery vehicle, PDV2, was superior to HHT in a saline delivery vehicle, SDVI, after oral administration.

**Table 4**

| Treatment | # of Tumors | Route | Dose (mg/kg) | Days to 4x (Ave ± SE) | T/C | Median | Delay |
|---|---|---|---|---|---|---|---|
| Untreated | 8 | - | -- | 7.1 ± 0.5 | 0.0 | 6.8 | 0.00 |
| HHT-SDV1 | 8 | IP | 4 | 7.5 ± 0.2 | 1.1 | 7.5 | 0.69 |
| HHT- SDV1 | 8 | PO | 4 | 8.1 ± 0.4 | 1.1 | 7.6 | 0.74 |
| HHT - PdV2 | 8 | PO | 4 | 8.9 ± 0.5 | 1.3 | 8.6 | 1.71 |

Table 5 shows results in the treatment of RIF-I tumors with viscous oral homoharringtonine formulations. The results in Table 5 show that the efficacy of HHT in the viscous protein delivery vehicle, PDVI. was superior to both viscous lipid and carbohydrate delivery vehicles (not claimed). Repeated administration of HHT in CDV2 enhanced anti-tumor efficacy.

**Table 5**

| Treatment | # of Tumors | Route | Dose (mg/kg) | Days to 4x (Ave ± SE) | T/C | Median | Delay |
|---|---|---|---|---|---|---|---|
| Untreated | 8 | - | -- | 7.1 ± 0.5 | 0.0 | 6.8 | 0.00 |
| HHT - PDV1 | 8 | PO | 4 | 9.2 ± 0.7 | 1.3 | 9.4 | 2.57 |
| HHT - LDV1 | 8 | PO | 4 | 8.6 ± 0.4 | 1.2 | 8.7 | 1.81 |
| HHT-LDV2 | 8 | PO | 4 | 8.0 ± 0.3 | 1.1 | 8.1 | 1.27 |
| HHT - CDV1 | 8 | PO | 4 | 7.9 ± 0.3 | 1.1 | 7.9 | 1.01 |

(continued)

| Treatment | # of Tumors | Route | Dose (mg/kg) | Days to 4x (Ave ± SE) | T/C | Median | Delay |
|---|---|---|---|---|---|---|---|
| HHT - CDV2 | 8 | PO | 4 | 7.7 ± 0.3 | 1.1 | 7.8 | 0.92 |
| HHT - CDV3 | 8 | PO | 4 | 7.8 ± 0.5 | 1.1 | 7.5 | 0.63 |
| HHT - CDV2 | 8 | PO | 4x3* | 9.9 ± 0.4 | 1.4 | 9.8 | 2.95 |
| * Formulation given on day 0, 1 and 2 | | | | | | | |

Table 6 shows results in the treatment of RIF-I tumors with a combination protein and lipid delivery vehicle. Combining a lipid and protein delivery vehicle did not reduce the efficacy of either system by itself.

**Table 6**

| Treatment | # of Tumors | Route | Dose (mg/K g) | Days to 4x (Ave ± SE) | T/C | Media n | Dela y |
|---|---|---|---|---|---|---|---|
| Untreated | 8 | - | -- | 5.4 ± 0.3 | 0.0 | 5.2 | 0.00 |
| HHT-SDV1 | 8 | PO | 4 | 6.3 ± 0.2 | 1.2 | 6.3 | 1.02 |
| Treatment | # of Tumors | Route | Dose (mg/k g) | Days to 4x (Ave ± SE) | T/C | Media n | Dela y |
| HHT-LDV1 | 8 | PO | 4 | 7.1 ± 1.0 | 1-3 | 6.2 | 0.95 |
| HHT-PDV1 | 7/8 | PO | 4 | 6.4 ± 0.3 | 1.2 | 6.1 | 0.88 |
| HHT-LDV1:PDV1 (1: 1) | 8 | PO | 4 | 7.2 ± 0.9 | 1.3 | 6.2 | 0.94 |

Homoharringtonine administered in a saline delivery vehicle shows similar efficacy in the RIF-I tumor model after oral and IP administration. Improvements in efficacy after oral delivery were seen with the use of protein and lipid. Developing an oral formulation for homoharringtonine utilizing a protein based excipient vehicle, whether viscous or not, improves the efficacy of orally administered homoharringtonine.

## Claims

1. An oral dosage form comprising a therapeutically effective amount of a cephalotaxine and a pharmaceutically acceptable carrier selected from the group consisting of protein, lipid or combinations thereof,
wherein said protein is selected from the group consisting of sodium caseinate, gelatin, casein, soy protein (isolated), brown algae protein, red algae protein, whey protein concentrate, bakers yeast extract and albumin;
wherein said lipid is selected from the group consisting of coconut oil (refined), oleic acid, soybean oil (hydrogenated), aluminum palmitate, dilauryl thiodipropionate, enzyme-modified lecithin, calcium stearate, enzyme-modified fats, glyceryl palmitostereate, lecithin mono- and diglycerides, rapeseed oil, and vegetable oil.

2. The oral dosage form of claim 1 in the form of a tablet, capsule, film, powder, granule, solution, solid or suspension

3. The oral dosage form of claim 1 or claim 2 wherein said cephalotaxine is homoharringtonine.

4. The oral dosage form of claim 1 further comprising an enteric coating.

5. The oral dosage form of any one of claims 1 to 4 further comprising a pharmaceutically acceptable excipient.

6. The oral dosage form of claim 1 in a unit dosage form.

7. The oral dosage form of claim 6 wherein said unit dosage form is selected from the group consisting of liquid, powder, tablet and capsule.

8. The oral dosage form of claim 7 wherein said liquid is selected from the group consisting of an emulsion and an aqueous solution.

## Patentansprüche

1. Orale Darreichungsform, umfassend eine therapeutisch wirksame Menge eines Cephalotaxins und einen pharmazeutisch akzeptablen Träger, ausgewählt aus der Gruppe, bestehend aus Protein, Lipid oder Kombinationen davon, wobei das Protein aus der Gruppe, bestehend aus Natriumkaseinat, Gelatine, Kasein, Sojaprotein (isoliert), Braunalgenprotein, Rotalgenprotein, Molkeproteinkonzentrat, Backhefeextrakt und Albumin ausgewählt ist;
wobei das Lipid aus der Gruppe, bestehend aus Kokosöl (raffiniert), Ölsäure, Sojaöl (hydriert), Aluminiumpalmitat, Dilaurylthiodipropionat, enzymmodifiziertem Lecithin, Calciumstearat, enzymmodifizierten Fetten, Glycerylpalmitostearat, Lecithin-Mono- und Diglyceriden, Rapsöl und Pflanzenöl ausgewählt ist.

2. Orale Darreichungsform nach Anspruch 1 in Form einer Tablette, Kapsel, eines Films, Pulvers, Granulats, einer Lösung, eines Feststoffs oder einer Suspension.

3. Orale Darreichungsform nach Anspruch 1 oder Anspruch 2, wobei das Cephalotaxin Homoharringtonin ist.

4. Orale Darreichungsform nach Anspruch 1, die weiter eine magensaftresistente Beschichtung umfasst.

5. Orale Darreichungsform nach einem der Ansprüche 1 bis 4, die weiter einen pharmazeutisch akzeptablen Exzipienten umfasst.

6. Orale Darreichungsform nach Anspruch 1 in einer Einzeldarreichungsform.

7. Orale Darreichungsform nach Anspruch 6, wobei die Einzeldarreichungsform aus der Gruppe, bestehend aus Flüssigkeit, Pulver, Tablette und Kapsel ausgewählt ist.

8. Orale Darreichungsform nach Anspruch 7, wobei die Flüssigkeit aus der Gruppe, bestehend aus einer Emulsion und einer wässrigen Lösung ausgewählt ist.

## Revendications

1. Forme posologique orale comprenant une quantité thérapeutiquement efficace d'une céphalotaxine et un support pharmaceutiquement acceptable choisi dans le groupe consistant en une protéine, un lipide ou des combinaisons de ceux-ci,
dans laquelle ladite protéine est choisie dans le groupe constitué par le caséinate de sodium, la gélatine, la caséine, une protéine de soja (isolée), une protéine d'algue brune, une protéine d'algue rouge, un concentré de protéines de lactosérum, un extrait de levure de boulangerie et l'albumine ;
dans laquelle ledit lipide est choisi dans le groupe constitué par l'huile de coco (raffinée), l'acide oléique, l'huile de soja (hydrogénée), le palmitate d'aluminium, le thiodipropionate de dilauryle, la lécithine modifiée par des enzymes, le stéarate de calcium, les graisses modifiées par des enzymes, le palmitostéarate de glycéryle, les mono-et diglycérides de lécithine, l'huile de colza, et l'huile végétale.

2. Forme posologique orale selon la revendication 1 sous la forme d'un comprimé, d'une capsule, d'un film, d'une poudre, d'un granulé, d'une solution, d'un solide ou d'une suspension.

3. Forme posologique orale selon la revendication 1 ou la revendication 2, dans laquelle ladite céphalotaxine est l'homoharringtonine.

4. Forme posologique orale selon la revendication 1, comprenant en outre un enrobage entérique.

5. Forme galénique orale selon l'une quelconque des revendications 1 à 4, comprenant un excipient pharmaceutiquement acceptable.

6. Forme posologique orale selon la revendication 1 sous une forme posologique unitaire.

7. Forme posologique orale selon la revendication 6, dans laquelle ladite forme posologique unitaire est choisie dans le groupe constitué par un liquide, une poudre, un comprimé et une capsule.

**8.** Forme posologique orale selon la revendication 7, dans laquelle ledit liquide est choisi dans le groupe constitué par une émulsion et une solution aqueuse.

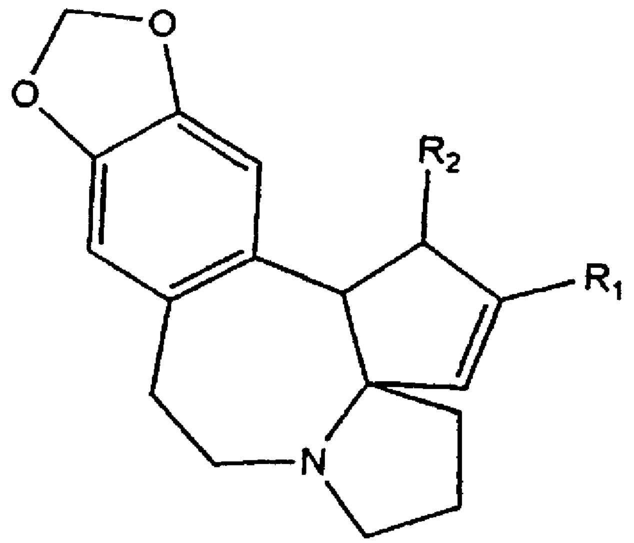

FIGURE 1

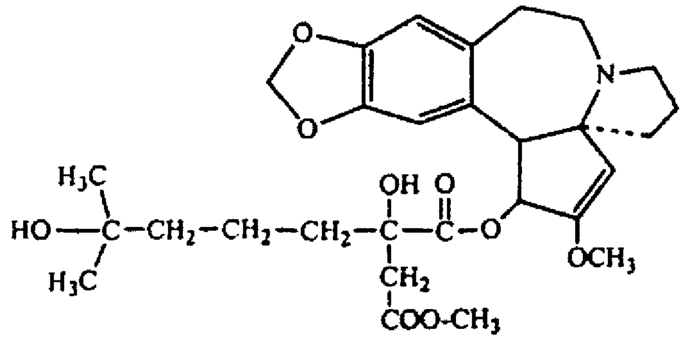

FIGURE 2

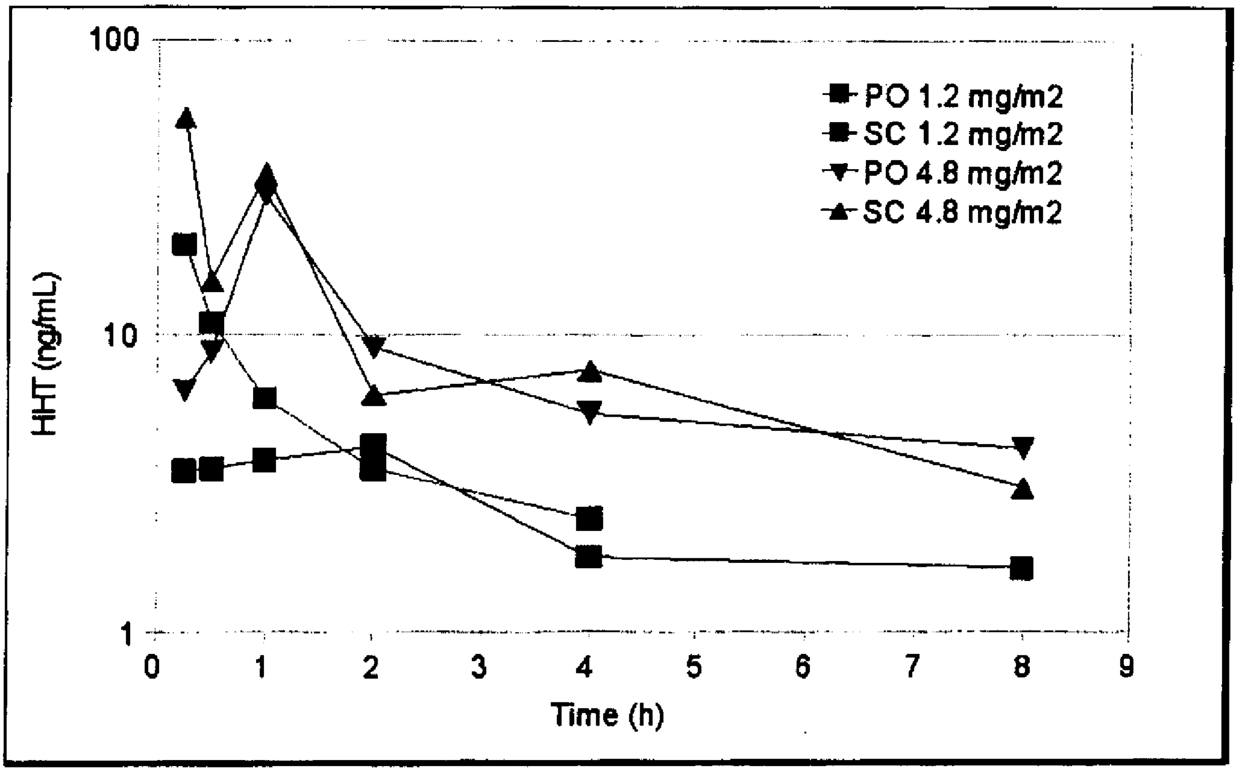
100
10
1
HHT (ng/mL)
0
1
2
3
4
5
6
7
8
9
Time (h)
PO 1.2 mg/m2
SC 1.2 mg/m2
PO 4.8 mg/m2
SC 4.8 mg/m2

**FIGURE 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004082565 A
- US 6926907 B
- US 6599529 B
- US 6365184 B
- US 6869615 B
- US 6184220 B
- US 6284269 B
- US 6682747 B
- US 6613354 B
- US 6740340 B
- US 6855702 B
- US 6605300 B
- US 4287221 A
- US 6861072 B
- US 6905708 B
- US 6723348 B
- US 6692771 B
- US 6365182 B
- US 6221392 B
- US 6899899 B
- US 7008640 B
- US 7014862 B
- US 4136145 A
- US 4713243 A
- US 5166233 A
- US 5700478 A
- US 5800832 A
- US 5948430 A
- US 6419903 B
- US 6177096 B
- US 6284264 B
- US 6596298 B
- US 6656493 B
- US 6709671 B
- US 6824829 B
- US 6923981 B
- US 20010046511 A
- US 20010022964 A
- US 20020131990 A
- US 20030107149 A
- US 20040151756 A
- US 20040241242 A
- US 20040247649 A
- US 20040258896 A
- US 20050184427 A
- US 20050196358 A
- US 20050075432 A
- US 20050037055 A
- US 092217 A


### Non-patent literature cited in the description


- **POWELL, R.G.** *J. Pharm Sci.,* 1972, vol. 61 (8), 1227-1230
- **FOLKMAN, J. ; SHING, Y.** *J. Biol. Chem.,* vol. 267 (16), 10931-10934
- **FOLKMAN, J. ; KLAGSBRUN, M.** *Science,* 1987, vol. 235, 442-447
- **FOLKMAN, J.** *Cancer Research,* 1986, vol. 46, 467-473
- **FOLKMAN, J.** *J. National Cancer Institute,* 1989, vol. 82, 4-6
- **WEIDNER. N. et al.** *The New England Journal of Medicine,* 1991, vol. 324 (1), 1-8
- **FOLKMAN, J.** Tumor Angiogenesis. *Adv. Cancer Res.,* 1974, vol. 19, 331-358
- **AUSPRUNK, D. H. ; FOLKMAN, J.** Migration and Proliferation of Endothelial Cells in Preformed and Newly Formed Blood Vessels During Tumor Angiogenesis. *Microvasc. Res.,* 1977, vol. 14, 53-65
- **WILKINSON-BERKA J. L. et al.** The Interaction Between the Renin-Angiotensin System and Vascular Endothelial Growth Factor in the Pathogenesis of Retinal Neovascularization in Diabetes. *J Vase Res.,* 2001, vol. 38 (6), 527-35
- **WALSH, D.A. ; PEARSON C.I.** Angiogenesis in the Pathogenesis of Inflammatory Joint and Lung Diseases. *Arthritis Res.,* 2001, vol. 3, 147-153
- **STORGARDL, C.M. et al.** Decreased Angiogenesis and Arthritic Disease in Rabbits Treated with an vβ3 Antagonist. *J Clin Invest,* 1999, vol. 3 (I), 47-54
- Remington: The Science and Practice of Pharmacy. Mack Publishing Co, 2006
- Handbook of Pharmaceutical Excipients. American Pharmaceutical Association, 2000
- Pharmaceutical Dosage Forms - Tablets. Marcel Dekker Inc, 1998
- Remington's Pharmaceutical Sciences. Mack Publishing